# EUROPEAN PATENT SPECIFICATION

(11) **EP 3 505 180 B1**
(45) Date of publication and mention of the grant of the patent: **03.08.2022**
(21) Application number: 19155435.1
(22) Date of filing: 27.09.2012
(51) Int. Cl.: A61K 38/39, A61K 31/728, A61K 8/64, A61Q 19/08, A61P 17/02, A61P 17/00, A61P 17/16, A61P 39/06

(54) **IN VIVO SYNTHESIS OF ELASTIC FIBER**
IN-VIVO-SYNTHESE EINER ELASTISCHEN FASER
SYNTHÈSE IN VIVO DE FIBRES ÉLASTIQUES

(30) Priority: 30.09.2011 AU 2011904041
(43) Date of publication of application: 03.07.2019
(62) Divisional of application: 12835454.5
(73) Proprietor: Allergan Pharmaceuticals International Limited, Dublin 17, D17 E400 (IE)
(72) Inventor: Mithieux, Suzanne Marie, Sydney, New South Wales 2006 (AU); Weiss, Anthony Steven, Sydney, New South Wales 2006 (AU)
(74) Representative: Nederlandsch Octrooibureau

(56) References cited:
- WO-A1-2008/058323
- WO-A1-2010/102337
- WO-A2-2009/034559
- US-A1- 2002 150 564

## Description

### Field of the invention

The invention relates to restoring or recreating elasticity in tissue, thereby improving the physical appearance and/or function of aged or injured tissue.

### Background of the invention

Ageing and tissue injury are associated with degeneration of the extracellular matrix leading to loss of tissue structure and/or function. Loosened skin, relaxed subcutaneous tissue, loss of density of the extracellular matrix, wrinkling, stretch marks and fibrosis are the physical manifestations of the degeneration. Depending on the relevant tissue, the loss of elastic function may manifest as decreased pulmonary or cardiac capacity or decreased compliance and/or resilience of various valves and sphincters.

About 20 years ago, the research effort sought to use the various molecules of the extracellular matrix in a range of clinical and cosmetic interventions for correcting loss of tissue structure and function. Key molecules of interest were those that are substrates of the relevant extracellular matrix fibers, namely collagen and elastin. Generally the approach was to use these biomaterials, either as implants or fillers to augment tissue appearance by filling tissue voids or by plumping or filling tissue, or to use these fibers as implants or fillers to improve defective function.

Elastin was considered by some as advantageous for this work because unlike collagen, it could be used to form elastic implants and fillers. The early work focussed on synthesis of recombinant forms of tropoelastin which would then be coacervated and chemically or enzymatically cross linked, either before or after delivery to an individual, so that an elastic implant or filler would be formed either *ex vivo* or *in vivo* for filling tissue voids or for augmenting or re-shaping tissue. See for example WO1994/14958; WO1999/03886; WO2000/04043. WO2010/102337 refers to the relevance of solids concentration in the formation of an injectable cross linked biomaterial.

Where enzymatic cross linking was used *in vivo,* recombinant or other exogenous lysyl oxidase was used. USSN 09/498,305 describes one approach to enzymatic cross linking of tropoelastin monomers *in vivo* by administration of a composition including exogenous lysyl oxidase and tropoelastin monomers.

Another approach to the formation of a material resembling certain characteristics of cross linked tropoelastin is disclosed in WO2008/058323 whereby an elastic material comprised of non cross linked tropoelastin is formed under alkaline conditions.

In each of the above examples, the exogenous tropoelastin and cross linking agent or alkaline conditions are utilised to drive the formation of the implant or filler. The time to formation of the elastic end product is a function of the concentration of tropoelastin, cross linking agent and relevant conditions, so that the end product results from a process that is acellular.

A number of other uses of tropoelastin were also contemplated including: (i) as a wound sealant (WO94/14958); (ii) as a delivery vehicle for active ingredients providing biodegradable or biodissociable slow release formulations (WO94/14958) (iii) as a binding reagent for GAGs (WO99/03886); (iv) for interfering with elastin deposition (WO99/03886); and (v) in wound sites, locations of tissue damage and remodelling where serine proteases are generally found (WO00/04043).

The early work suggested that multiple forms of tropoelastin could be used for any one of the above applications. See for example WO94/14958 which relates to a synthetic form of human tropoelastin including domain 26A. WO94/14958 describes mammalian and avian forms for use in pharmaceutical compositions; WO99/03886 which relates to a number of synthetic forms of human tropoelastin, including those lacking domain 26A, C-terminal domain and others. WO99/03886 describes human and non human forms for use in pharmaceutical applications. A particular form, SHELδ26A is discussed with reference to a lack of GAG binding activity; and WO00/04043 which relates to forms of tropoelastin having reduced susceptibility to serine proteases, specifically thrombin, plasmin, kallikrein, gelatinases A and B and matrix metallo elastase. WO00/04043 describes the relevant forms of tropoelastin having reduced susceptibility, (referred to as "reduced tropoelastin derivative") useful in these applications including partial and full length forms and xenogeneic forms.

In each example of this early work, while an implant with elastic properties could be provided to tissue, the nature of the implant and its elastic properties was not suggestive of that normally ascribed to the tissue. For example, the elastic properties imparted by a filler or implant as described in this early work to dermal or subcutaneous tissue could be seen to be clearly different to the normal elasticity of that tissue. To put in other words, while elasticity could be imparted to a tissue by the implantation of a material with properties that include elasticity, a return to a physical appearance or function resembling normal could not.

In hindsight this outcome is perhaps unsurprising as more recent work over the last 5 to 10 years has revealed that the elastic profile of a given tissue results from a complex process involving multiple factors in addition to lysyl oxidase and tropoelastin known as 'elastogenesis'. Elastogenesis is generally understood as referring to a physiological process occurring from late fetal life to early post natal life whereby elastic fiber is created *de novo* by cells including fibroblasts, smooth muscle cells and the like from tropoelastin monomers and other relevant factors. Starting with a common set of factors, a relevant tissue provides for tissue specific interplay of these factors resulting in a synthesis, organisation and distribution of elastic fiber that is natural to the relevant tissue and from which the elastic profile of the tissue arises (Cleary E.G and Gibson M.A. Elastic Tissue, Elastin and Elastin Associated Microfibrils in Extracellular Matrix Vol 2 Molecular Components and Interactions (Ed Comper W.D. ) Harwood Academic Publishers 1996 p95). What has become clear is that this organisation, and the concomitant profile cannot be re-created simply by cross linking exogenous tropoelastin with exogenous lysyl oxidase either *ex vivo* or *in vivo* as proposed by the early work.

The initiation of a process that is like elastogenesis (i.e. one whereby the tissue synthesises an elastic fiber *de novo* from a common set of factors) in adult tissue is a desirable goal because it is believed that such a process would restore an elastic profile to a tissue. For example, an elastic profile of an aged tissue could be restored so that the profile of the tissue resembles that of a younger tissue. Unfortunately the goal remains elusive, principally because there is negligible formation of elastic fiber *de novo* in an adult. Although elastic fiber repair may occur in some cardiovascular and pulmonary diseases, the integrity and organisation of elastic fiber arising from repair mechanisms is unlike that arising from elastogenesis. (Akhtar et al. 2010 J. Biol. Chem. 285: 37396-37404).

This problem has been intensively studied by a number of research groups over the last decade (Huang R et al., Inhibition of versican synthesis by antisense alters smooth muscle cell phenotype and induces elastic fiber formation in vitro and in neointima after vessel injury. Circ Res. 2006 Feb 17; 98(3):370-7; Hwang JY et al., Retrovirally mediated overexpression of glycosaminoglycan-deficient biglycan in arterial smooth muscle cells induces tropoelastin synthesis and elastic fiber formation in vitro and in neointimae after vascular injury. Am J Pathol. 2008 Dec;173(6):1919-28.; Albertine KH et al., Chronic lung disease in preterm lambs: effect of daily vitamin A treatment on alveolarization. Am J Physiol Lung Cell Mol Physiol. 2010 Jul 299(1):L59-72; Mitts TF et al., Aldosterone and mineralocorticoid receptor antagonists modulate elastin and collagen deposition in human skin. J Invest Dermatol. 2010 Oct,130(10):2396-406, Sohm B et al., Evaluation of the efficacy of a dill extract in vitro and in vivo. Int J Cosmet Sci. 2011 Apr;33(2):157-63; Cenizo Vet al., LOXL as a target to increase the elastin content in adult skin: a dill extract induces the LOXL gene expression. Exp Dermatol. 2006 Aug;15(8):574-81). The widely considered hypothesis for explaining the absence of elastic fiber formation *de novo* in an adult is that adult cells or the relevant tissue in which they are contained lack one or more of the necessary factors and processes required for elastogenesis (Shifren A & Mecham R.P. The stumbling block in lung repair of emphysema: elastic fiber assembly. Proc Am Thorac Soc Vol 3 p 428-433 2006). According to the hypothesis, the provision of synthetic tropoelastin to adult tissue should not enable an adult cell to synthesise elastic fiber from the synthetic tropoelastin.

Current research has focussed on understanding the mechanisms and factors underpinning elastogenesis in early life and to determine whether these are present in adult life (Wagenseil JE & Mecham RP. New insights into elastic fiber assembly. Birth Defects Res C Embryo Today. 2007 Dec;81(4):229-40.)

It is generally thought that shortly after tropoelastin protein expression it coacervates into an assembly of spheres of about 200-300nm which then further coalesce into particles of about one micron. These particles then assemble along the length of microfibrils in the extracellular matrix thereby forming elastic fiber (Kozel BA et al., Elastic fiber formation: a dynamic view of extracellular matrix assembly using timer reporters. J Cell Physiol. 2006 Apr;207(1):87-96). The involvement of a range of additional factors in this process continues to be explored.

In vitro studies of the various molecular steps have tended to examine human and non human tropoelastin substrates and a range of different tropoelastin isoforms (Davidson JM et al., Regulation of elastin synthesis in pathological states. Ciba Found Symp. 1995;192:81-94; discussion 94-9). Through this work it has been revealed that at least 34 different molecules are associated with elastic fibers, although only some of these have been shown to be structurally involved in fiber production. These include tropoelastin, fibrillin-1, fibrillin-2, lysyl oxidase, Lysyl oxidase -like-1 (LOXL1), emilin, fibulin-4 and fibulin -5 (Chen et al. 2009 J. Biochem 423: 79-89). One group considers LOXL1, a member of the LOX family as being the key missing molecule in certain adult tissue (see US2004/0258676, US2004/0253220 and US20100040710). Other groups identify fibulin 4 and other molecules, either through interaction with lysyl oxidase or other molecules (Yanagisawa H & Davis EC. Unraveling the mechanism of elastic fiber assembly: The roles of short fibulins. Int J Biochem Cell Biol. 2010 Jul;42(7):1084-93).

In summary, while the picture regarding the interplay of factors in elastogenesis is not yet complete, the current research indicates that adult cells and tissues do not complete a process that is like elastogenesis because they lack one or more factors. It follows that the provision of tropoelastin alone to adult tissue should not in itself be sufficient to restore the elastic profile of the tissue, because without the relevant factors required for elastogenesis, the tissue cannot utilise the tropoelastin to form an elastic fiber.

There remains a need to restore or recreate an elastic profile of a tissue, or to minimise the degeneration of an elastic profile of a tissue.

There is a need to improve the elastic profile of an aged tissue so that it more closely resembles the profile of the tissue at an earlier stage of life.

There is a need to improve the physical appearance of aged tissue, including photo -aged tissue, for example to minimise loosened skin, relaxed subcutaneous tissue, loss of density of the extracellular matrix, wrinkling and stretch marks.

There is also a need to improve the elastic profile in scarred or fibrotic tissue so that the profile more closely resembles the profile of the relevant tissue containing the scar or fibrotic tissue before tissue injury.

There is also a need to provide improved elastic function in aged or injured tissue that more closely resembles the elastic function of the relevant tissue at an earlier stage of life or prior to injury.

The above mentioned needs are distinct from those addressed by implants or fillers and use of same to fill tissue with cross linked tropoelastin, as in the relevant prior art *supra.*

### Summary of the invention

The invention seeks to address one or more of the above mentioned needs. The invention is as defined in the appended claims.

In one embodiment provides a method of restoring an elastic profile of a tissue of an individual including:
- providing an individual having a tissue in which an elastic profile is to be restored;
- administering tropoelastin to the individual according to a treatment regime that has been selected to maintain the administered tropoelastin in the tissue for a period of time that enables factors expressed in the tissue for formation of an elastic fiber to engage with the administered tropoelastin for synthesis of elastic fiber therefrom;
- thereby restoring or recreating the elastic profile of the tissue of the individual.

In another embodiment there is provided a method of minimising the degeneration of an elastic profile of a tissue of an individual including:
- providing an individual having a tissue in which degradation of an elastic profile is to be minimised;
- administering tropoelastin to the individual according to a treatment regime that has been selected to maintain the administered tropoelastin in the tissue for a period of time that enables factors expressed in the tissue for formation of an elastic fiber to engage with the administered tropoelastin for synthesis of elastic fiber therefrom;
- thereby minimising the degeneration of an elastic profile of a tissue of an individual.

In another embodiment there is provided a method of improving the elastic profile of an aged tissue so that it more closely resembles the profile of the tissue at an earlier stage of life, including:
- providing an individual having a tissue in which an elastic profile is to be improved;
- administering tropoelastin to the individual according to a treatment regime that has been selected to maintain the administered tropoelastin in the tissue for a period of time that enables factors expressed in the tissue for formation of an elastic fiber to engage with the administered tropoelastin for synthesis of elastic fiber therefrom;
- thereby improving the elastic profile of the aged tissue so that it more closely resembles the profile of the tissue at an earlier stage of life.

In another embodiment there is provided a method of improving the physical appearance of aged tissue, including:
- providing an individual having a tissue in which a physical appearance is to be improved;
- administering tropoelastin to the individual according to a treatment regime that has been selected to maintain the administered tropoelastin in the tissue for a period of time that enables factors expressed in the tissue for formation of an elastic fiber to engage with the administered tropoelastin for synthesis of elastic fiber therefrom;
- thereby improving the physical appearance of aged tissue.

In another embodiment there is provided a method of improving the elastic profile in scarred or fibrotic tissue so that the profile more closely resembles the profile of the relevant tissue containing the scar or fibrotic tissue before tissue injury including:
- providing an individual having a scarred or fibrotic tissue;
- administering tropoelastin to the individual according to a treatment regime that has been selected to maintain the administered tropoelastin in the tissue for a period of time that enables factors expressed in the tissue for formation of an elastic fiber to engage with the administered tropoelastin for synthesis of elastic fiber therefrom;
- thereby improving the elastic profile in scarred or fibrotic tissue.

In another embodiment there is provided a method of improving the elastic function in aged or injured tissue that more closely resembles the elastic function of the relevant tissue at an earlier stage of life or prior to injury including:
- providing an individual having an aged or injured tissue;
- administering tropoelastin to the individual according to a treatment regime that has been selected to maintain the administered tropoelastin in the tissue for a period of time that enables factors expressed in the tissue for formation of an elastic fiber to engage with the administered tropoelastin for synthesis of elastic fiber therefrom;
- thereby improving the elastic function in aged or injured tissue.

In another embodiment there is provided a method of providing elasticity to the skin of an individual, preferably for providing thickness to the skin of an individual while maintaining or improving the elasticity of the skin of the individual, the method including the following steps:
- providing an individual;
- defining a treatment area on the skin of the individual, wherein the treatment area is an area of skin in which elasticity is to be provided;
- injecting a tropoelastin composition within the treatment area so as to establish an amount of tropoelastin within the treatment area that is increased relative to skin outside the treatment area;
- maintaining the amount of tropoelastin in the treatment area for a pre-determined period of time, thereby providing elasticity to the skin of an individual.

In another embodiment there is provided a tropoelastin composition as described herein for use in one or more of the following applications:
- restoring an elastic profile of a tissue of an individual
- minimising the degeneration of an elastic profile of a tissue of an individual
- improving the elastic profile of an aged tissue so that it more closely resembles the profile of the tissue at an earlier stage of life
- improving the physical appearance of aged tissue
- improving the elastic profile in scarred or fibrotic tissue so that the profile more closely resembles the profile of the relevant tissue containing the scar or fibrotic tissue before tissue injury
- improving the elastic function in aged or injured tissue that more closely resembles the elastic function of the relevant tissue at an earlier stage of life or prior to injury.

### Brief description of the drawings

Figure 1: Fluorescent images showing elastin networks formed 7 days after 250 µg/ml tropoelastin addition to cultured human fibroblasts sourced from different age groups. A. Neonatal primary male (NHF 8-9-09); B. 10 year old male (GM3348); C. 31 year old male burn patient (230209A); D. 51 year old male (142BR); E. 92 year old male (AG04064). Elastin network deposition is in green, cell nuclei in blue.
Figure 2: Fluorescent images showing elastin deposition 7 days after tropoelastin addition to cultured pig and rabbit fibroblasts. A. 10 week primary pig skin fibroblasts; B. Adult rabbit skin fibroblasts. Elastin deposition is green, cell nuclei are blue.
Figure 3: Fluorescent images showing elastin fiber formation by primary airway smooth muscle cells 7 days subsequent to the addition of tropoelastin. A. airway smooth muscle cells (3785). B. another source of airway smooth muscle cells (3791). Elastin deposition is green, cell nuclei are blue.
Figure 4: Fluorescent images showing elastin fiber formation by primary neonatal human fibroblasts 7 days subsequent to the addition of tropoelastin or derivatives. A. No tropoelastin; B. full length tropoelastin; C. human skin elastin peptides; D. RKRK deletion; E. RGDS substitution. F. Advanced Biomatrix tropoelastin. Elastin deposition is green, cell nuclei are blue.
Figure 5: Fluorescent images showing elastin fiber formation by primary neonatal human fibroblasts 7 days subsequent to the addition of 125 µg/ml tropoelastin in the absence (A) and presence (B) of 50 µM blebbistatin. Elastin deposition is green, cell nuclei are blue.
Figure 6: Fluorescent images showing extent of elastin network formation by primary neonatal human fibroblasts following repeated tropoelastin additions. A. Cells; B. Cells + tropoelastin addition on Day 10; C. Cells + tropoelastin additions on Day 10 and Day 17; D. Cells + tropoelastin additions on Day 10, Day 17 and Day 24. All samples were fixed for imaging on Day 31. Elastin deposition is green, cell nuclei are blue.
Figure 7: Autofluorescing mature elastin fibers. (A) fibroblasts with no added tropoelastin, (B) fibroblasts with added tropoelastin.
Figure 8: AFM analysis of dermal human fibroblast cultures. Images represent culture topography overlaid with modulus channel. (A) fibroblasts with no added tropoelastin, (B) fibroblasts with added tropoelastin.
Figure 9: Elastic fiber formation by human neonatal dermal fibroblasts. Tropoelastin was added 12 days post-seeding and samples stained with DAPI, anti-elastin mouse antibody and FITC-conjugated anti-mouse.
Figure 10: Inhibition of lysyl oxidase prevents elastic fiber formation. Elastin fiber formation in the presence of the lysyl oxidase inhibitor BAPN. Samples are stained with DAPI, anti-elastin mouse antibody and FITC-conjugated anti-mouse.
Figure 11: Super resolution microscopy images of tropoelastin spherules within a human dermal fibroblast culture. (A) Scale bar is 1 micron. (B) Scale bar is 2 microns.
Figure 12: TEM images of human dermal fibroblast culture 3 days after tropoelastin was added.
Figure 13: Verhoeff-Van Gieson (VVG) stained sections of week 2 biopsies. VVG staining for elastin in dermal cross sections in pig skin 2 weeks subsequent to treatment of a full thickness wound with tropoelastin containing constructs. Test A is cross-linked collagen template cross-linked in the presence of 10% tropoelastin. Test B is cross-linked collagen template applied on top of a tropoelastin matrix cross-linked to a modified HA. Images are contrasted with normal pig skin and the Control which is cross-linked collagen template.
Figure 14: Skin biopsy sections taken from subjects treated with either RVL or elastin based formulations in the upper arm dermis. (A) Skin treated with RVL shows dermal collagen fibers stretched apart by unstained RVL material which makes the skin stiffer and lumpy. (B) Skin treated with tropoelastin based formulations results in dermal collagen fibers separated by implant material which is stained by VVG from blue to purple to black indicating the implant material is remodeled into mature elastin fibers.

### Detailed description of the embodiments

It is believed that the key findings of the invention arise from a novel assay system developed by the inventors and exemplified in the Examples herein. The assay system uses adult human cells to form elastic fiber *in vitro.* The system can be manipulated so as to enable dissection of each step of elastic fiber formation, and to identify components and processes required for elastic fiber formation.

This assay system has revealed a pathway of elastic fiber synthesis unlike that previously understood before the invention. A key finding is that fiber formation is much more dependent on cell interaction than previously thought.

A key finding is that the system does not result in substantial or otherwise significant synthesis of elastic fiber unless exogenous tropoelastin monomer is added to the system. This points to the importance of tropoelastin in the synthesis of elastic fiber *in vivo.*

Further to this, the system demonstrates that the elastic fiber formation does not occur efficiently if the system uses human tropoelastin monomers with non-human cells.

Further the monomers are generally required to take the form of one or more naturally occurring isoforms. While the monomers may be synthesised recombinantly, it has been found that recombinant forms that have a sequence or structure that does not exist in human physiology do not enable efficient elastic fiber formation, although fiber formation remains possible to some extent provided that the sequence difference between endogenous and exogenous tropoelastin is not lower than about 65% homology.

Further to this, repeat administration of tropoelastin to the system demonstrates an ongoing capacity to form elastic fiber, indicating that the tropoelastin is the limiting factor to elastic fiber formation.

It is believed that the use of human adult cells and naturally occurring human tropoelastin isoforms distinguishes the assay system from others (see for example Sato F et al., Distinct steps of cross-linking, self-association, and maturation of tropoelastin are necessary for elastic fiber formation. J Mol Biol. 2007 Jun 8;369(3):841-51) and it is probable that this is why these relevant research groups have not understood that human adult cells do have potential for synthesis of elastic fiber in a process resembling elastogenesis, provided that the cells are exposed to tropoelastin.

In further studies a clinical trial exemplified herein establishes the importance of maintaining tropoelastin in tissue for enough time for cells to engage with the tropoelastin. This may be achieved by establishing and maintaining a level of tropoelastin in an area of tissue to be treated for a select period of time so that the treated area has a level of tropoelastin greater than an untreated area. It is believed that, provided that the tropoelastin persists in the tissue for a long enough period of time required for engagement of cells, or where the tissue has few cells, for recruitment of and engagement of cells, an elastogenesis-like process may occur in adult tissue resulting in formation of fiber and a restoration of elastic profile in the tissue. Exemplary time periods for persistence or maintenance of tropoelastin in tissue are discussed further below.

It will be understood that the elastic fiber formed in accordance with the invention may have the same molecular structure as that observed in nature, although in some embodiments the molecular and/or physical structure of the fiber may be different. In certain embodiments the elastic fiber may have a physical structure distinct from that in the treated tissue, whilst still achieving the aims of the invention.

In particular embodiments the elastin that is synthesised according to the methods of the invention integrates with tissues, cells and/or extracellular matrix, thereby restoring or recreating elastic profile, improving physical appearance or achieving other clinical endpoints. In these embodiments, the synthesised elastin may have a different physical or molecular structure as compared with elastic fiber normally observed in the tissue, and the obtaining of an end point may result from an interaction or engagement between the elastin and the other components of the relevant tissue. The interaction or engagement may ostensibly model those processes normally seen between elastic fiber and tissue elements in the relevant tissue.

In one embodiment, the elastic fiber formed according to the invention is provided in a hydrated form, thereby imbuing the fiber with elastic potential.

The studies forming the basis of this invention demonstrate that a restoration or recreation of elastic profile is possible in adult tissue because adult cells such as fibroblasts, smooth muscle cells and the like have an elastogenic potential; that is a potential to engage in a process that is like elastogenesis and that therefore returns a relevant elastic profile to the tissue. Further the potential is realised provided that the adult cells are provided with species and potentially tissue relevant isoforms of tropoelastin monomer. In addition, it has been shown by the inventors that recombinant human tropoelastin that contains substantial levels of impurities does not result in efficient formation of elastin fiber. In certain embodiments the tropoelastin has a specified degree of purity with respect to the amount of tropoelastin in a composition for administration, as compared with amounts of other proteins or molecules in the composition. In one embodiment, the tropoelastin is in a composition that has at least 75% purity, preferably 85% purity, more preferably more than 90, or 95% purity. It will be understood that in certain embodiments the tropoelastin may be provided in the form of a composition that consists of, or consists essentially of tropoelastin, preferably a full length isoform of tropoelastin. Finally, cells are unable to utilize tropoelastin to form elastic fiber if the tropoelastin has already been substantially intra-molecularly cross linked.

According to the invention, the treatment regime is one which maintains tropoelastin within a defined treatment area of a tissue for a sufficient time within which cells may engage and utilize the administered tropoelastin to form elastic fiber. An appropriate regime may involve more than a single administration of tropoelastin monomers, or more than administration of unadulterated monomer, because it is believed that tropoelastin monomers have a half life within a defined treatment area of tissue which is generally less than that required for the relevant cells to form elastic fiber. In more detail it is believed that tropoelastin monomers that do not engage with cells are either metabolised in a treatment area, or disperse from a treatment area. It follows that without selection of an appropriate treatment regime, an administered tropoelastin may be ostensibly depleted from a defined treatment area before it can be utilized by a cell to form an elastic fiber.

One step in the treatment regimes described further below may include a single administration of tropoelastin where the site to which the tropoelastin is administered is known to have a significant number of cells. The knowledge of cell number or density may be derived from prior histological knowledge of the tissue. Alternatively, the site of administration may have been prior treated with a treatment for inducing cell proliferation or recruitment to the treatment site.

A number of treatment regimes could be adopted to maintain administered tropoelastin in tissue for the required time in a treatment area. These are broadly as follows:
( i) administration of tropoelastin in a sustained release formulation that gradually releases tropoelastin over a period of time.

The sustained release of tropoelastin at the required tissue site may be achieved by incorporation of the tropoelastin into a non-degradable or a degradable delivery vehicle. A number of such sustained release approaches could be employed by one skilled in the art. Preferably a degradable sustained release formulation is employed to avoid the need for removal of the vehicle once the tropoelastin has been delivered. Such delivery vehicles may be composed of polymers such as Polylactide (PLA) and Poly (Lactide-co-Glycolide) (PLGA). Other sustained delivery vehicles may include polymers formed from polysaccharides such as hyaluronic acid, xanthan gum or chitosan. In addition, in certain embodiments the delivery vehicle may be chemically modified to bind the tropoelastin by ionic or covalent bonds into the implant such that the tropoelastin is only released as the implant is degraded.

In certain embodiments the tropoelastin is released at the required treatment site for a period of between 1 to 90 days. In certain embodiments the tropoelastin may be released at the required treatment site for between 1 to 180 days. In certain embodiments the tropoelastin may be formulated so that it is released only after a delay following application of the implant such as from 10 to 90 days or from 10 to 180 days. Other appropriate tropoelastin delivery times include 1 to 30 days, 1 to 60 days, 10 to 60 days, 30 to 60 days, 30 to 180 days, or for 1 to >180 days.

The amount and concentration of tropoelastin to be delivered is dependent on both the area and volume of tissue to be treated, the typical endogenous levels of elastin present in the tissue normally; and, the level of elastin fiber synthesis required. Typically tropoelastin will be delivered to the tissue in an amount of 1µg to 1mg per each cm³ of tissue. For skin this may be calculated as 1µg to 1mg of cm². Other amounts which may be delivered include 0.1 µg to 10mg per each cm³ of tissue, 1mg to 20mg per each cm³ of tissue, or 1mg to 100mg per cm³ of tissue. In certain embodiments the amounts delivered may be less than 0.1µg or more than 100mg per cm³ of tissue. The concentration of tropoelastin in the implants to be applied to the treated site may vary to enable the required amounts of tropoelastin to be delivered. In certain embodiments the concentration of tropoelastin in the implants may vary from 1µg/ml to 100mg/ml. In certain embodiments the tropoelastin concentration in the product will be between 0.5mg/ml and 200mg/ml, 1mg/ml and 50mg/ml, 5mg/ml and 50mg/ml or 1mg/ml and 25mg/ml.

The tropoelastin incorporated in the formulation should be substantially equivalent to an isoform of tropoelastin which occurs naturally in the tissue to be treated. In addition, the tropoelastin should be provided in a form which is substantially devoid of impurities. Fragments of tropoelastin, i.e. truncated forms of a tropoelastin isoform that arise unintentionally through tropoelastin manufacture may be regarded as an impurity in this context. In certain embodiments tropoelastin incorporated into the treatment formulation will be at least 65% of the length of the relevant full length tropoelastin isoform, more preferably 80% of the relevant full length tropoelastin isoform. In other embodiments the tropoelastin will be more than 85%, more than 90% or more than 95% full length. As described herein, certain sequences in tropoelastin are more critical than others, for example, the efficiency of fiber formation increases where the final C-terminal sequence of amino acids in tropoelastin of about 4 residues have homology or identity with the tropoelastin sequence that is endogenous to the relevant tissue.

Additional components may also be included in the formulation to assist in the activation of cells required in the tissue to form the elastic fiber. For example for the treatment of skin, additional components may be incorporated into the formulation which assist in the recruitment or proliferation of fibroblast cells at the treatment site. Such components include the epidermal growth factor family, transforming growth factor beta family, fibroblast growth factor family, vascular endothelial growth factor, granulocyte macrophage colony stimulating factor, platelet-derived growth factor, connective tissue growth factor, interleukin family, and tumor necrosis factor-α family.

In certain embodiments the treatment may also include the delivery of cells to the treatment site with the tropoelastin. By way of example for the treatment of skin, fibroblasts may be included in the treatment formulation or procedure to aid the synthesis of elastic fiber at the treatment site. The fibroblast cells may be sourced from an allogeneic source such as neonatal foreskin or sourced by biopsy of a non-visible skin site (e.g. behind the ear) and used as an autologous treatment.
(ii) administration of tropoelastin in which protease susceptible regions have been removed or blocked from enzymes present in tissue

The tropoelastin used in the treatment may be modified to reduce protease degradation. For example protein species may be selected as described in WO2000/04043 to the extent that they remain substantially full length tropoelastin species naturally found in the tissue to be treated. Alternatively, the treatment formulations may incorporate protease inhibitors or molecules which block signalling pathways known to increase protease expression. Such molecules include serine protease inhibitors, matrix metalloproteinase inhibitors, galactosides such as lactose, inhibitory antibodies and small molecule inhibitors of elastin signalling
(iii) repeated administration of tropoelastin at pre-defined time points.

In certain embodiments, to ensure the tropoelastin is delivered in a form which can be utilised by cells as a substrate for the construction of elastic fiber and remain at the treatment site for a sufficient period of time for this to occur, the treatment is applied to the site on repeated occasions.

In certain embodiments each tissue site to be treated will receive the three treatments of the product, from 1 to 24, or 2 to 12 or 3 to 6 weeks apart. The treatment may consist of multiple injections across the area to be treated, each approximately 10mm apart in a grid formation. The treatment may be administered using a fine gauge needle, such as a 27G, 29G, 30G or 31G. The needle may be inserted into the tissue with consideration to the angle and orientation of the bevel, the depth of injection, and the quantity of material to be administered. The treatment may be injected into the tissue as a bolus, with for example a volume of 10-100µl, 10-50ul, preferably 20 to 30uL of product implanted at each injection site. After completion of each injection, the needle may be slowly withdrawn. When all implants have been completed the treated site may be gently massaged if required to enable the implant material to conform to the contour of the surrounding tissues. The number of treatments, the period between treatments and the amount of tropoelastin delivered at each treatment site will be adjusted based on the tissue area to be treated and the level of elasticity to be restored.

Any one of these approaches could be implemented singularly or in combination, thereby increasing the persistence of tropoelastin in tissue.

In each of the approaches it will be recognised that the step of administration is an invasive procedure having potential to cause reversible tissue or cell injury and the initiation of the various inflammatory cascades that arise in response to such injury. The inventors recognise that this type of physical treatment may be applied so as to provide conditions for reversible cell injury, as such conditions are likely to stimulate fibroblast activation and/or proliferation. It is important that the physical treatment is not sufficient to induce fibrosis.

As discussed herein, the considerations that guide a selection of a particular treatment regime include the nature of the tissue, the extent of degradation or degeneration of elastin profile, and the outcome desired. Again, a critical aspect of the invention is that cells are given opportunity to form, repair or synthesise elastic fiber from the tropoelastin provided to them. There is more opportunity where because of sustained release, protection from degradation or continuous supply, tropoelastin effectively persists in tissue for a longer period of time. Generally the greater the loss of elastic profile and the more acellular the tissue, the more appropriate it is that a treatment regime should provide for persistence of tropoelastin in tissue for a longer period of time.

In more detail, a shorter persistence time may be appropriate where the objective is to improve the physical appearance of younger skin as compared with such an improvement to older skin. Here, repeated administrations of tropoelastin at pre-defined time points according to (iii) and/or (i) above may be more appropriate.

A longer persistence time may be required where tissue is scarred or fibrotic and essentially acellular. Here it will be important to leave sufficient time for chemotaxis of cells into the relevant tissue. A regime according to (ii) and/or (i) may be more appropriate.

As mentioned, the outcome is also a relevant consideration guiding the selection of an appropriate regime. Where the outcome is to increase or to improve elastic function, a much longer persistence time enabling cells to build the required elastic fiber array specific to the function may be required. Here a sustained release form may be more appropriate as in (i) above.

Some examples of considerations relevant to the selection of appropriate treatment regimes are discussed in more detail below:
(i) Improving physical appearance of skin.
(ii) Increasing elastin content of fibrotic and scarred tissue.
(iii) Improving elasticity of cartilaginous or vasculature.

Nearly all mammalian elastic tissues have an elastin profile that arises from the elastic fibers contained within them. As each different elastic tissue has a different function, it follows that the elastic profile is not the same from tissue to tissue. For example, the resilience of left side vasculature to blood flow is not the same as the resilience of bronchial tissue to inhaled air. The following table describes examples of tissue to which the invention is directed and how the elastic profile of each may be measured and expressed:

| **Tissue** | **Relevant elastic characteristic forming the elastic profile** | **How elasticity is measured** |
|---|---|---|
| Skin | Young's modulus | Cutometer |
| | Skin elasticity as measured by the Cutometer or Torque measurements | Ballistometry |
| | is typically described as: | Torque measurements |
| | Ue (elastic stretch in °) | |
| | Uv (viscoelastic stretch in °) | |
| | Ur (elastic recovery in °) | |
| | Measurements usually include Ur/Ue or Ur/(Ue+Uv) | |
| | Ur/Ue varies for skin site and thickness and depending on the measuring device. Typically a result of 0.5-0.8 is obtained for normal skin. As one gets older this lowers and the range may become, e.g., 0.35 - 0.6. Sun damaged skin or other skin damage may similarly impact the elasticity. A successful treatment may improve this Ur/Ue ratio after treatment by increasing both Ur and Ue. Care must be taken when interpreting Ur/Ue ratios as the skin may appear more elastic (higher Ur/Ue ratio) when in fact it is just stiffer (Ue has reduced significantly with no change to or even reduced Ur). | |
| | For example in scarred tissue the skin will be less elastic and the total stretch of the skin (Ue + Uv) will be dominated by Uv. In this scenario the Ur/Ue may seem quite high because the skin site has minimal | |
| | stretch ability. A successful treatment in this scenario may simply increase the Ue component of total stretch (Ue+Uv). | |
| Bronchial tissue | Alveolar elastin content | Spirometer |
| Blood vessel | Intima and media elastin content | Vessel compliance and response to systeole/diastole |
| Bladder | Radial elastin in bladder wall | Volume and retention |
| Elastic ligament | Organisation of elastic fibers | Tissue flex, extensibility and return around ligament site |
| Sphincter | Spatial elastin distribution to support muscle function | Retention and extension |
| Nucleus pulposis | Movement, compression and recoil to restore and maintain disc shape | Spinal measurement device |

Typically the individual treated according to the invention is a human.

Preferably, the tissue is skin tissue, especially tissue in skin tissue in an individual of at least 20 years, preferably 20 to 50 years of age, more preferably 30 to 60 years of age.

The skin tissue may be characterised by a breakage or fragmentation of elastic fibers at the junction of the dermis and epidermis.

The skin tissue may be photo -aged tissue.

The skin tissue may present with one or more of the following features: loosened skin, relaxed subcutaneous tissue, loss of density of the extracellular matrix, wrinkling and stretch marks.

The skin tissue is preferably located on the face, neck or upper or lower limb.

Preferably the tissue does not contain a wound at the time of commencement of the treatment regime. It is possible that at the completion of administration of tropoelastin according to a selected treatment regime that there is minor wounding of the tissue, as for example where administration is by injection or other physical manipulation of the skin.

Where the individual is human, the tropoelastin has the sequence of a tropoelastin isoform that is expressed in a human. In this embodiment, the isoform may be selected from the group consisting of SHEL (see WO1994/14958) and SHELδ26A (see WO1999/03886) and protease resistant derivatives of these isoforms (see WO2000/0403).

Typically the tropoelastin isoform is SHELδ26A where the tissue is human skin tissue.

The tropoelastin isoform may be provided in the form of a composition that is adapted for a sustained release of the tropoelastin in the tissue. Where the tissue is human skin tissue, it is preferred that the composition includes SHELδ26A and a component for sustained release of the tropoelastin from the composition selected from the group consisting of hyaluronan, glycosaminoglycans, collagen type I.

Typically the composition for administration including tropoelastin does not contain exogenous factors for elastic fiber formation, especially lysyl oxidase.

In certain embodiments the tropoelastin is provided according to a treatment regime in a substantially monomeric form.

In certain embodiments the tropoelastin is provided according to a treatment regime in a form substantially lacking intra-molecular cross-links.

In certain embodiments the tropoelastin is provided according to a treatment regime in a composition that consists of tropoelastin and a solvent for the tropoelastin, such as an aqueous solution. Preferably the tropoelastin is SHELδ26A.

In certain embodiments the tropoelastin is provided according to a treatment regime in a composition that consists essentially of tropoelastin. In one embodiment the tropoelastin is SHELδ26A.

In certain embodiments, the treatment includes tropoelastin and a hyaluronic acid.

In certain embodiments, the tropoelastin in the composition may be cross linked to derivatised hyaluronic acid (HA). The cross-linking of the tropoelastin to a molecule such as hyaluronic acid may help to maintain the tropoelastin at the implant site according to the current invention. The composition may have from 5 to 100mg/ml tropoelastin + 0.1% to 2% HA cross-linker, preferably from 10 to 50 mg/ml tropoelastin and 0.25% to 1% HA cross-linker. Suitable formulations for the invention may include from 10 to 30mg/ml tropoelastin cross-linked to from 0.25% to 1% HA cross-linker.

Importantly, the cross-linking of tropoelastin to polysaccharide such as hyaluronic acid may not result in, or involve intramolecular tropoelastin cross links, such as those that occur with lysyl oxidase. In more detail, if the hyaluronic acid is dissolved by hyaluronidase (a skin enzyme), the tropoelastin may then be released in monomeric form.

In certain embodiments, the treatment may involve compounds that increase the utilisation of tropoelastin. Examples include:
- diclofenac - an anti-inflammatory (and associated with reduction in actinic keratoses e.g. see Solaraze)
- Lys'lastine - to promote elastagenesis
- amino acids Gly, Val, Ala, Pro - corresponding to 75% of tropoelastin residues
- Vitamins C, E - Vitamin C assists new collagen formation and both are antioxidants
- sunscreen - limits sun-induced proteolysis
- chemical enhancers - assist transfer of components across stratum corneum
- pH adjusted in a moisturising emollient - to deliver pH for skin; moisturising is relevant to older skin

Where the tissue is skin, typically the treatment regime includes administration of tropoelastin at defined time points. At any one time point, there may be concurrent administration of tropoelastin.

Preferably the tropoelastin is administered by injection.

Where the tissue is skin, it is preferred that the tropoelastin is administered to the dermis.

In certain embodiments the treatment regime may additionally include the topical application of substances capable of augmenting the formation of elastic fiber. Such substances would be well known to those skilled in the art and may include but are not limited to a dill extract to stimulate lysyl oxidase expression (Cenizo et al 2006 Exp. Dermatol. 15:574-81); and, copper and/or zinc based creams to reduce elastic fiber breakdown (Mahoney et al 2009 Exp. Dermatol.18:205-211).

In one embodiment there is provided a method of providing elasticity to the skin of an individual, the method including the following steps:
- providing an individual;
- defining a treatment area on the skin of the individual, wherein the treatment area is an area of skin in which elasticity is to be provided;
- injecting a tropoelastin composition within the treatment area so as to establish an amount of tropoelastin within the treatment area that is increased relative to skin outside the treatment area;
- maintaining the amount of tropoelastin in the treatment area for a pre-determined period of time, thereby providing elasticity to the skin of an individual.

As described in the examples below, the method enables one to increase the thickness of skin while maintaining or improving skin elasticity. The method also enables improvements in skin elasticity, or restoration or recreation of elastic profile while retaining smoothness (i.e. avoiding lumpiness) and natural appearance of skin.

Typically the individual is an adult individual who has lost skin condition, as described herein. For example, the treatment area of skin may be characterised by photo -aging, loosened skin, relaxed subcutaneous tissue, loss of density of the extracellular matrix, wrinkling and stretch marks. The adult may be from 20 to 70 years of age, for example from 20 to 35 years of age or from 40 to 70 years of age.

As discussed herein, the skin that is preferably treated according to the invention may be located on the face, neck, or upper or lower limb. The treatment area may comprise all or part of the skin at the relevant location. For example, where the skin is located on the upper limb, the treatment area may comprise all of the upper limb, or part of it, for example the medial surface of the upper limb. Where the skin is located on the face, the treatment area may comprise all or part of skin about a cheek, eyelid, chin etc.

According to the invention, a treatment area is an area of skin in which elastic profile is suboptimal and/or requires improvement or restoration. This area may be defined in any number of ways known to the skilled worker. The simplest of these is to demarcate the area of skin requiring treatment from skin in which treatment is not required by indicating the limits or boundaries of the area to be treated. This may be done for example using a marker, indicator, guide or character that distinguishes the area to be treated from the area where treatment is not required, for example a marker that selectively identifies an area to be treated, or that selectively identifies an area where treatment is not required. In one embodiment, the area to be treated may be defined by identifying one or more coordinates that relevantly establish the boundary of the treatment area.

Having defined a treatment area, the tropoelastin composition may be injected intradermally into skin located within the treatment area. The purpose of the injection is to establish or provide an amount of tropoelastin to the treatment area that is not normally present in the treatment area. In this context, the amount of tropoelastin established in the treatment area is greater than the amount of tropoelastin in an adjacent or neighbouring area of skin located outside the treatment area.

The composition may be injected mid to deep dermis depending on where the treatment area is located. For example, deeper injections may be more appropriate for treatment areas where the skin is thicker such as the cheeks of the face than for treatment areas where the skin is thinner such as the neck, décolletage or around the eyes.

It will be understood that in some instances the target outcome may be achieved by implantation in the hypodermis and recruiting elastogenic cells to the site of the implantation or injection.

The volume of composition that is delivered is partly dependent on the location of the skin to be treated. Larger volumes are more appropriate or possible where the skin is located on a limb or neck, than on the face. The volumes of each single injection may range from 10 to 100uL, preferably about 20 to 50 uL. The overall volume of the treatment given will depend on the number of injections provided which in turn is dependent on the size of the skin area to be treated and the distance determined to be appropriate between each injection site.

In one particularly preferred form of the invention, a desired amount of tropoelastin is maintained in the treatment area for a pre-determined period of time, by repeated injection of tropoelastin to the treatment area. This ostensibly creates a continuous supply of tropoelastin to tissue in the treatment area so that the treatment area retains a threshold level of tropoelastin for in situ elastic fiber formation that is not found outside the treatment area. It is believed that over the course of a treatment (discussed below) this increases the likelihood of engagement of cells and factors with injected tropoelastin, thereby enabling elastic fiber formation.

In certain embodiments, the treatment is administered by injection of the tropoelastin composition into the mid to deep dermis by fine needle injection. The injection may be made using a hypodermic needle with a gauge of 25G, preferably, 27G or less, more preferably 30G or 31G. The injection may be made using a single syringe and needle by manual application of the treatment to the skin.

In certain embodiments, a single treatment may include multiple injections into a treatment area. Where each treatment requires multiple injections, these may be spaced from 1mm to 3cm apart.

In certain embodiments the injection may be made using a device which enables automated injection into the skin dermis such as a Mesotherapy gun, or an assisted injection device such as the artiste injection device (http://www.nordsonmicromedics.com/se/google/en/artiste-assisted-injection-system.html) or the anteis injection device (http://www.anteis.com/AestheticDermatology/injectionsystem.php). In certain embodiments the syringe or automated injection device may be used with an adaptor to enable multiple needles to be attached so that more than one injection can be applied at a time. In certain embodiments the treatment may be applied using a solid needle system such as a dermal roller, or dermapen needling system (e.g. as described by Kalluri, H. et al 2011, AAPS Journal 13:473-4841).

There may be a period of about 3 to 168 days between each treatment. Typical periods between each treatment may include 3 to 7 days, 3 to 21 days, 14 to 28 days, 21 to 84 days, and 3 to 84 days. There may be 1 to 24, or 3 to 6 treatments in total. Generally the period of treatment is no more than about 1 year, preferably from 3 weeks to 6 months, preferably about 1 to 3 months.

Preferred sites of treatment include those near, about, within or adjacent to cheeks, the eyes, neck, décolletage, hands, scarred tissue, stretch marks.

As used herein, except where the context requires otherwise, the term "comprise" and variations of the term, such as "comprising", "comprises" and "comprised", are not intended to exclude further additives, components, integers or steps.

Further aspects of the present invention and further embodiments of the aspects described in the preceding paragraphs will become apparent from the following description, given by way of example and with reference to the accompanying drawings.

It will be understood that the invention disclosed and defined in this specification extends to all alternative combinations of two or more of the individual features mentioned or evident from the text or drawings. All of these different combinations constitute various alternative aspects of the invention.

### Examples

### Example 1 In vitro assay system for elastic fiber synthesis

### Materials and Methods

a) Cells

| Cell code | Cell type | Age of donor | Source |
|---|---|---|---|
| NHF8909 | Primary human skin fibroblasts | Neonatal | University of Queensland, Australia |
| GM3348 | Human skin fibroblasts | 10 yo | Coriell Research Institute, NJ, USA |
| 230209A | Primary human skin fibroblasts (burns patient) | 31 yo | Anzac Research Institute, Australia |
| 142BR | Human skin fibroblasts | 51 yo | European Collection of Cell Cultures |
| AG04064 | Human skin fibroblasts | 92 yo | Coriell Research Institute, NJ, USA |
| Pig 10-10 | Primary porcine skin fibroblasts | 10 weeks | University of Queensland, Australia |
| RAB-9 | Rabbit skin fibroblasts | Adult | European Collection of Cell Cultures |
| 3785 | Primary human airway smooth muscle cells (lung transplant patient) | 28 yo | Woolcock Institute of Medical Research, Australia |
| 3791 | Primary human airway smooth muscle cells (lung resection patient) | 59 yo | Woolcock Institute of Medical Research, Australia |

b) Cell culture

Cells were cultured in Dulbecco's Modified Eagle Medium High Glucose (DMEM; Invitrogen) containing 10% fetal bovine serum (FBS; Invitrogen) and 1% (v/v) penicillin/streptomycin (Invitrogen). Media were changed every 2-3 days. Cells were incubated at 37°C and 5% CO₂. To assess the capacity of cells to form elastin fibers 1 x 10⁵ cells were seeded onto glass coverslips in 12 well culture plates. Ten to 17 days post-seeding full-length tropoelastin (Elastagen), or an alternative elastin-derived protein, in PBS was filter-sterilized and added to the cell cultures. Alternative elastin-derived proteins included human skin elastin peptides (Elastin Products Company; HSP72), a C-terminal tropoelastin deletion construct ΔRKRK (Weiss lab) and a C-terminal tropoelastin substitution construct containing RGDS (Weiss lab). Fiber formation was also assessed in the presence of 50 µM blebbistatin (Sigma). For experiments assessing the effect of repeated tropoelastin additions the protein was added 10, 17 and 24 days post-seeding. Cell matrix thickness was determined by averaging the number of 0.41 µm z slices required to image from the uppermost nuclei to the bottom of the sample in ten randomly chosen fields of view.

At set time points after tropoelastin addition, cells were fixed with either 3% (w/v) formaldehyde or 4% (w/v) paraformaldehyde for 20 min and quenched with 0.2 M glycine. The cells were incubated with 0.2% (v/v) Triton X-100 for 6 min, blocked with 5% bovine serum albumin at 4°C overnight, and stained with 1:500 BA4 (Sigma) mouse anti-elastin primary antibody for 1.5 hr and 1:100 anti-mouse IgG-FITC secondary antibody (Sigma) for 1 hr. The coverslips were then mounted onto glass slides with ProLong Gold antifade reagent with DAPI (Invitrogen).

### c) Fluorescence imaging

Samples were visualized with an Olympus FluoView FV1000 confocal microscope. Images shown here were constructed by z-stack projection.

### Results and Discussion

### a) Elastin fiber formation by human skin fibroblasts sourced from different age groups

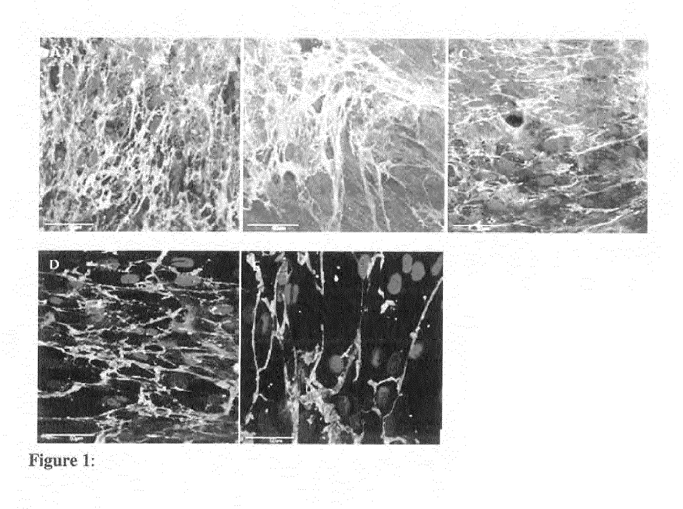

We assessed the capacity of human skin fibroblasts to form elastin fibers and networks following the addition of tropoelastin (SHELδ26A (i.e synthetic human elastin that does not contain domain 26A)). Figure 1 shows elastin formation 7 days post 250 µg/ml tropoelastin addition to skin fibroblasts sourced from neonatal, 10, 31, 51 and 92 year old donors. All cell lines demonstrated elastin fiber formation. No elastin formation was seen in control cell cultures where tropoelastin was not added (data not shown). Younger donor cells proliferated more extensively as shown by the increased number of nuclei (blue). Younger donor cells created extensive elastin networks when tropoelastin was added. Older donor cells were still capable of creating substantial elastin fibers from added tropoelastin however the network was sparser (Figure 1).

### b) Elastin fiber formation by animal cells

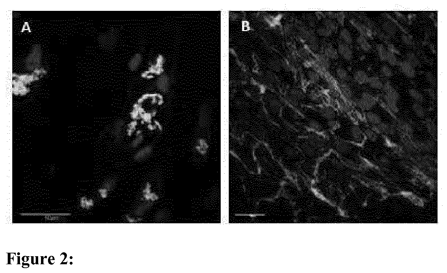

The capacity of pig (Pig 10-10) and rabbit (RAB-9) skin fibroblasts to form elastin fibers and networks following the addition of 250 µg/ml tropoelastin was assessed. As shown in Figure 2 each of these animal cells deposited tropoelastin into the matrix. However, only the rabbit cells were capable of producing an elastin network. Tropoelastin amino acid sequence differences between human and animal species may account for the lower efficiency, varied utilization of tropoelastin by animal cells (Figure 2).

### c) Elastin fiber formation by airway smooth muscle cells

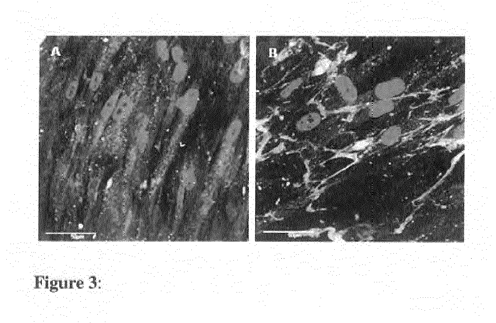

We assessed the capacity of primary human airway smooth muscles cells sourced from diseased lungs to form elastin fibers following the addition of tropoelastin. Figure 3 shows elastin formation 7 days post 250 µg/ml tropoelastin addition. These cells differed in the extent of fiber formation: from a minimal amount of tropoelastin spherule deposition to an elastin fiber network (Figure 3). The results demonstrate that the smooth muscle cells, like fibroblasts observed in Figure 1, have capacity for formation of elastic fiber from exogenous tropoelastin, as would smooth muscle cells from other tissues, such as vasculature.

### d) Elastin fiber formation when tropoelastin derivatives are used

We assessed the capacity of primary human neonatal fibroblasts (NHF8909) to form elastin networks using three alternative elastin-derived proteins. These proteins were elastin skin peptides prepared by enzymatic hydrolysis of human adult skin elastin with human sputum elastase and two tropoelastin isoforms. Tropoelastin contains a motif GRKRK at its C-terminus which we have shown directs cell binding to αᵥβ₃ integrin. In the tropoelastin isoform ΔRKRK the RKRK sequence of this motif has been removed. In the isoform +RGDS the RKRK sequence has been removed and replaced with the canonical cell binding domain RGDS. In all cases 125 µg/ml protein was added to primary human neonatal skin fibroblasts 12 days post-seeding.

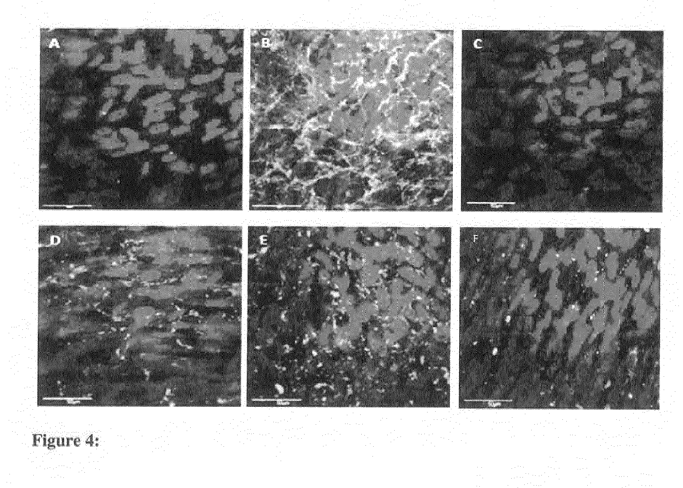

Figure 4 demonstrates the resulting elastin networks. Elastin fiber formation was observed when full length tropoelastin was added to the cultures. In contrast, fiber formation was significantly impaired when tropoelastin derivatives were added to the cultures. There was no deposition of skin elastin peptides into the matrix. Spherule rather than fiber deposition of each of the ΔRKRK and +RGDS forms was observed.

### e) Elastin fiber formation when cellular contractile forces are impaired

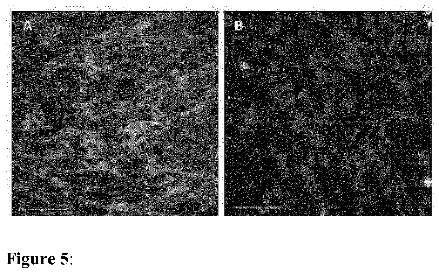

We investigated the requirement for cellular contractile forces in elastin fiber formation by adding blebbistatin to the cell culture at the same time as tropoelastin was added. Blebbistatin is an inhibitor of non-muscle myosin II that alters cellular contractile forces and cell migration. Figure 5 shows that elastin fiber formation is substantially impaired in the presence of blebbistatin.

### f) Elastin fiber formation following repeated tropoelastin additions

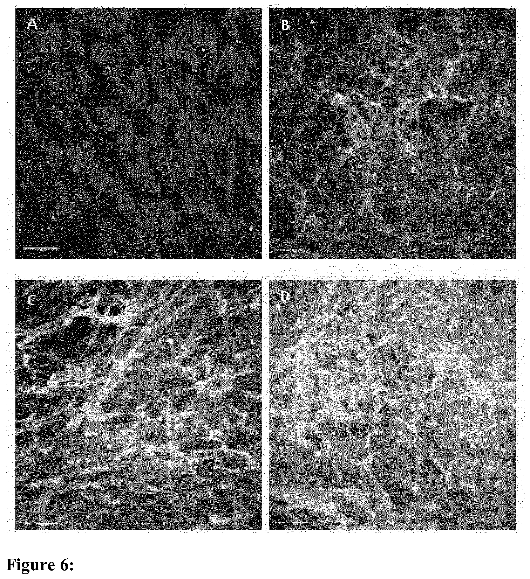

We assessed the capacity of primary neonatal human skin fibroblasts (NHF8909) to form elastin networks from repeated additions of tropoelastin. Tropoelastin (250 µg/ml) was added to cultures 10 days, 10 and 17 days, and 10, 17 and 24 days post seeding. All samples were fixed 31 days post seeding. Figure 6 shows that elastin network formation increased substantially with repeated tropoelastin treatments. This resulted in an increase in the cell-matrix thickness where a sample without added tropoelastin 31 days post seeding was 13.4 ± 2.2µm thick, samples with one and two tropoelastin additions were 15.3 ± 1.2 µm and 16.9 ± 0.8 µm thick respectively and a sample with three tropoelastin additions was 19.0 ± 2.2µm thick.

### g) Elasticity of in vitro formed fiber

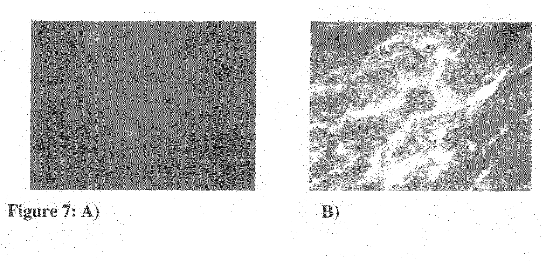

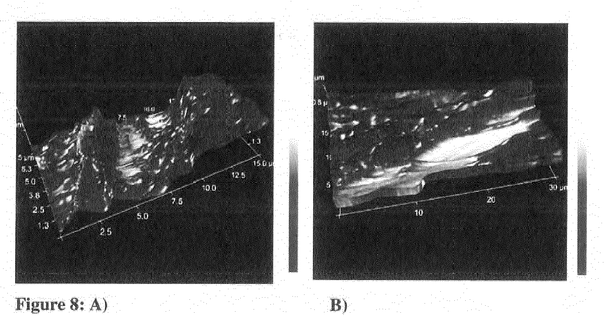

Human dermal fibroblasts were seeded on WillCo glass bottom dishes at a density of 20,000 cells/cm² in DMEM (Invitrogen, 11995) supplemented with 10% (vol/vol) fetal bovine serum and 1% (vol/vol) penicillin/streptomycin. At 12 days after seeding, 250 µg/mL tropoelastin in PBS was added to the fibroblast cultures. Culture media was changed every 2 days. At 19 days post seeding samples were analyzed with a BioScope Catalyst Atomic Force Microscope. The intrinsic autofluorescence of mature elastin fibers was used to indicate their position within the culture. Time matched control samples with no tropoelastin addition did not display autofluorescence (Fig 7). Topography/Elastic Modulus mapping demonstrated changed culture elasticity (Fig 8, yellow areas) following tropoelastin addition as evidenced by a dominant region of intercellular material with a Young's Modulus of ~600kPa, consistent with the formation of elastic fibers. Unpurified natural elastin has a Young's Modulus of ~600 kPa.

### h) Time course for elastic fiber formation

Human dermal fibroblasts were seeded on glass coverslips at a density of 20,000 cells/cm2 in DMEM supplemented with 10% (vol/vol) fetal bovine serum and 1% (vol/vol) penicillin/streptomycin. At 10-12 days after seeding, 250 µg/mL tropoelastin in PBS was added to the fibroblast cultures. Culture media was changed every 2 days. At set days, generally 1, 3 and 7, after tropoelastin addition, cells were fixed with 4% (wt/vol) paraformaldehyde for 20 min and quenched with 0.2 M glycine. The cells were incubated with 0.2% (vol/vol) Triton X-100 for 6 min, blocked with 5% bovine serum albumin at 4°C overnight, and stained with 1: 500 BA4 mouse anti-elastin antibody for 1.5 h and 1:100 anti-mouse IgG-FITC antibody for 1 h. The coverslips were then mounted onto glass slides with ProLong Gold antifade reagent with DAPI. Samples were visualized using an Olympus FluoView FV1000 confocal microscope. Z stacks were taken and converted to compressed projection images.

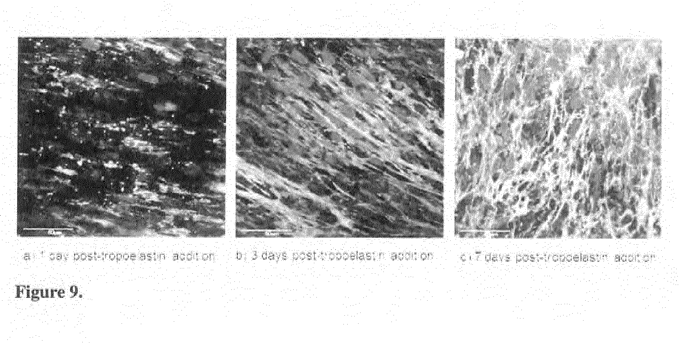

This in vitro cell culture model system shows that following tropoelastin addition the protein is deposited into the ECM as spherules (Fig 9a). Subsequent fiber formation is initially aligned in the direction of cells (Fig 9b) before generating an extensive branched elastic network (Fig 9c).

### i) Involvement of lysyl oxidase

The effect of the lysyl oxidase inhibitor BAPN on elastic fiber formation in this system was studied.

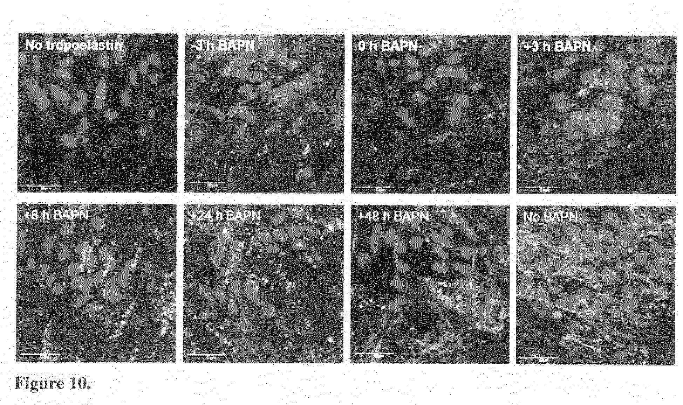

Dermal human fibroblasts were grown for 12 days prior to tropoelastin addition as described. Cells were cultured for a further 72 hours after tropoelastin addition. BAPN was added at various time points relative to tropoelastin addition. Samples were stained for elastin and nuclei as described above. Inclusion of the BAPN permits some spherule deposition into the ECM but prevents fiber formation (Fig 10), demonstrating that the cells utilize lysyl oxidase during the formation of elastic fiber from the tropoelastin.

### j) Alignment of spherules

Super resolution microscopy was used to further investigate elastic fiber formation in an in vitro model system. Human dermal fibroblasts were cultured and fixed 3 days after tropoelastin addition as described. Cells were stained with 1\500 BA4 mouse anti-elastin antibody for 1.5 h and 1\100 anti-mouse IgG-AlexaFluor 488 antibody for 1 h. Samples were visualized with a Leica SP5 cwSTED microscope.

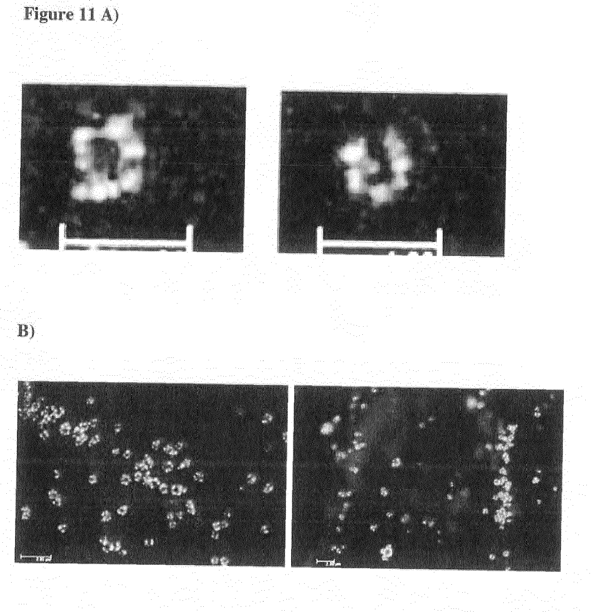

Aligning spherules are found 3 days after adding tropoelastin to a 12 day old dermal fibroblast culture (Fig 11). The spherules show punctate decorations with the antibody. The average spherule diameter is 605 ± 97 nm.

### k) Processing of spherules

Transmission electron microscopy was also used to further investigate elastic fiber formation in an in vitro model system essentially as described. Samples were processed 3 days after tropoelastin addition to a 12 day old dermal fibroblast culture. Cells grown on elastin were post-fixed with 2% glutaraldehyde in PBS buffer for 1hr at 4°C and were next post-fixed with 0.1% osmium tetroxide for 10 min in the dark at 4°C and immediately washed twice with distilled water for 5 min each. Subsequently, the samples were dehydrated through a gradient series of ethanol for 10 min each (i.e., 70, 80 and 90% and two times 100%). Infiltration of the sample with Epon (resin) was achieved with the following mixtures and incubation times: 25% Epon in ethanol for 4 hrs, 50% Epon in ethanol overnight and two changes of 100% Epon for 8 hr each at room temperature. When resin infiltration was complete, the sample was embedded using the double polymerization method of Kobayashi K., et al. (2012). The resulting block faces containing the embedded cells were trimmed and ultrathin sections generated via an ultramicrotome (Leica, Ultracut-7), yielding sections of approximately 70 nm that were mounted on 200 mesh copper grids. Sections were stained with 2% aqueous uranyl acetate and Reynolds's lead citrate for 10 min each, and were washed thoroughly with water in between steps to minimize stain deposits. The sections were imaged using a JEOL 2100 TEM (JEOL, Japan) at 200 kV.

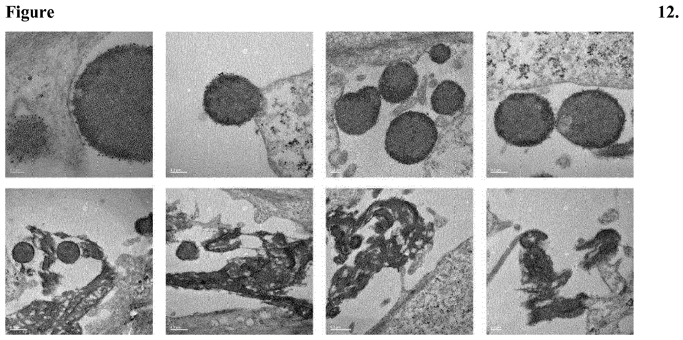

Three distinct elastin-containing structures are seen (Fig 12):
(1) Spherules surrounded by a dense shell with an average diameter of 615 ± 153 nm. These spherules are in direct contact with the cells.
(2) Spherules that ruptured, spilling out their contents.
(3) Elastic masses formed from coalescing ruptured spherules.

The close association of the elastic material with cells and cell projections suggests that mechanical forces disrupts the spherules.

### 1) Animal model

The effect of tropoelastin containing dermal templates together with thin split skin grafting on elastin fiber formation was examined. Two pigs were used in the study. The following skin substitutes were applied at day 0.
1. Control: cross-linked collagen template alone
2. Test A: cross-linked collagen template cross-linked in the presence of 10% tropoelastin
3. Test B: cross-linked collagen template applied on top of a tropoelastin matrix cross-linked to a modified HA

On Day 0 four excisional wounds (5 cm diameter) were created on the upper back of each pig. Two wounds from one side were covered with Control. One wound from the other side was treated with test A and the other wound was treated with test B. On Day 7 (week 1) dressings were changed for all wounds. On Day 14 (week 2) 4mm biopsies a few mm away from the edge of the wounds were collected. On day 21 (week 3) thin split skin grafting was carried out on all wounds with dressing changes. On Day 28 (week 4) dressings were changed for all wounds. On Day 35 (week 5) the animals were euthanized and wound tissue and normal skin was collected. Biopsies were fixed in formalin and embedded in paraffin.

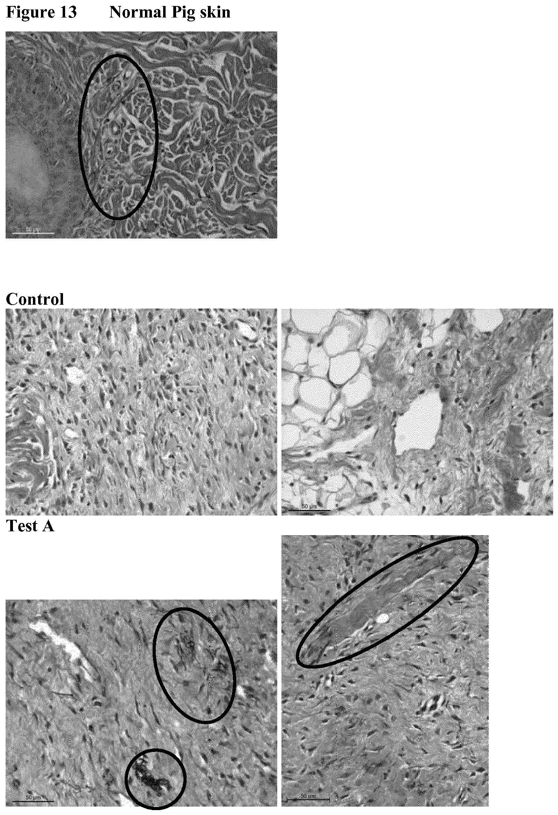

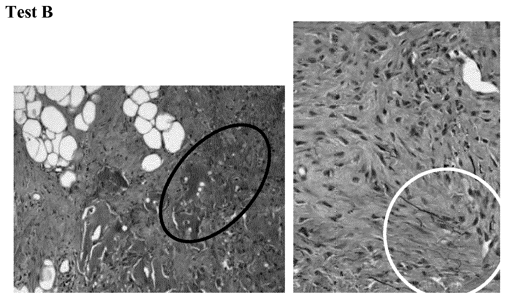

Elastin fiber formation was assessed by Verhoeff van Gieson staining of sections (Figure 13) which renders elastin fibers purple/black in color. Elastin fibers are seen surrounding a hair follicle in normal pig skin (circled).

Short, sporadically observed fibers were occasionally seen in the dermis of the control samples.

After biopsy of Test A samples, tissue from the wound site displayed de novo elastin in the form of fibers and collections of fibers (e.g. areas highlighted with black circles).

After biopsy of Test B samples, tissue from the wound site displayed persistent tropoelastin matrix cross-linked with modified HA (e.g. area highlighted with black circle), and de novo elastin fiber formation (e.g. area highlighted with white circle).

### Example 2. Clinical Study to Assess the Treatment of Human Skin using an Elastin Injectable Skin Rejuvenation Product.

### Methods:

A clinical study was undertaken using a formulation of tropoelastin lightly cross-linked with a derivatised hyaluronic acid (as described in PCT/AU2011/001503, in particular Example 3 and Example 6) compared to Restylane Vital Light (RVL - 12mg/ml hyaluronic acid cross-linked with BDDE, Q-Med, Australia). Participants were treated on the skin on the inside of the upper arm by implanting the product into the dermis by fine needle injection. The upper arm was chosen for the study as this is an area of skin which is not typically exposed to sun light and so presents as healthy undamaged skin tissue. The study aimed to assess the impact of the products on skin thickness and texture including elasticity and to gather subjective patient feedback on the appearance, naturalness and smoothness of the treated skin site.

Healthy subjects were recruited to the study and following a screening period, sixteen subjects who met the entry requirements were enrolled and randomly assigned to receive treatment with one of a range of tropoelastin formulations (ELAPR002: 10 - 30mg/ml tropoelastin cross-linked to a derivatised hyaluronic acid) on one arm plus the control Restylane Vital Light (RVL - 12mg/ml hyaluronic acid cross-linked with BDDE) on the other arm. All subjects received three such treatments at the same treatment site, 3 weeks apart. Each treatment consisted of multiple injections of 20-30ul of product delivered using a 30Gx¼" needle, each approximately 1cm apart in a grid formation over the area upper arm.

At each visit, subjects were asked questions relating to the smoothness, naturalness and appearance of the skin at the treated site and asked to provide feedback via a Visual Analogue Scale (VAS) by marking a line on a scale from 0-100 (0 being not very smooth, natural or poor appearance, and 100 being very smooth, natural and good appearance). Measurements of skin elasticity and skin thickness were made using a Dermal Torque Meter (DTM) and skin calipers, respectively. Histopathology of biopsy sections was undertaken at 3 months and 6 months to assess the persistence of the implants and the levels of elastin content at the treatment sites by Verhoff Van Giesen (VVG) staining.

### Results:

### Histopathology Analysis of Implant Sites:

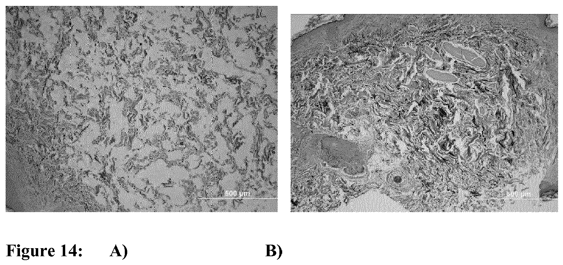

Skin sites assessed by VVG revealed that skin areas treated with RVL showed dermal changes including dermal collagen fibers being stretched and spread apart by the implant material as shown by the unstained extracellular spaces which dominate Figure 14A. By contrast, skin areas treated with ELAPR002 showed the implant material integrating with the skin tissue with evidence of remodeling of the implant material into elastin as evidenced by the implant material transitioning from blue, to purple to black under VVG staining. It is clear therefore that the administration of tropoelastin has provided for *in situ* assembly and deposition of elastic fiber much like that observed in elastogenesis (Figure 14B).

### Measurements of Skin Thickness & Lumpiness

Skin thickness at the treatment sites was measured by the investigating clinician using skin calipers. Table 1 shows mean skin thickness measurements for sites treated with RVL and ELAPR formulations at baseline and 3 months. The increase in skin thickness was found to be significant for both RVL and elastin formulations (p<0.001).

**Table 1: Skin thickness measurements**

| Time / Product | RVL | ELAPR002i | ELAPR002ii |
|---|---|---|---|
| Baseline Skin Thickness (mm) | 1.66 | 1.51 | 1.6 |
| 3 months Skin Thickness (mm) | 2.55 | 1.95 | 2.28 |

Of the sixteen patients in the study, all sixteen arms treated with RVL presented with lumps which were visible and could be felt by the investigator at 3 months. By contrast only 1 of the 16 arms treated with ELAPR002 formulations presented with any lumps at 3 months. As such, the skin thickness measurements for RVL are largely a measurement of the lumps of RVL in the skin, whereas the measurements of the ELAPR002 treated sites more accurately reflect an increase in general skin thickness across the treated area.

### Measurements of Skin Elasticity

Measurements of the elastic stretch of the skin, Ue, were taken from the treated skin sites using the DTM at each assessment visit throughout the period of the clinical study.

The mean Ue scores at base line and 6 months are provided in Table 2 for RVL and ELAPR002i. As can be seen from the data in the table, skin sites treated with RVL revealed a decreasing capability of elastic stretch (reduced Ue after treatment), indicating that the increased skin thickness resulting from treatment with RVL is making the skin stiffer. In contrast, skin areas treated with the tropoelastin implants maintained the capability of elastic stretch (Ue remains relatively stable), indicating that the increased skin thickness is achieved whilst maintaining the skin's elastic properties.

**Table 2: Skin Elastic Stretch (Ue)**

| Time/Product | RVL | ELAPR002i |
|---|---|---|
| Mean Ue at baseline (°) | 5.07 | 4.93 |
| Mean Ue at six months (°) | 3.85 | 4.55 |

### Patient Assessments

The mean scores from the patient visual analogue assessment of the treated skin area smoothness, naturalness and appearance are provided in Table 3 for skin sites treated with RVL and ELAPR002ii. The data shows that patients rated the skin sites treated with tropoelastin formulations highly for smoothness, naturalness and appearance compared to those treated with RVL (higher scores representing a positive assessment).

| | Comfort/ Formulation | RVL | | ELAPR002ii |
|---|---|---|---|---|
| | Mean skin smoothness baseline | 74.9 | | 68.6 |
| | Mean skin smoothness 6 months | 48.9 | | 81.1 |
| | Mean skin naturalness baseline | 84.8 | | 79.8 |
| | Mean skin naturalness 6 months | 51.9 | | 83.1 |
| | Mean skin appearance baseline | 82.9 | | 74.9 |
| | Mean skin appearance 6 months | 43.6 | | 82.0 |

## Claims

1. A non-therapeutic method of improving the appearance of skin in an individual, the method including the following treatment steps:
- providing an individual;
- defining a treatment area on the skin of the individual, wherein the treatment area is an area of skin requiring improvement of appearance
- injecting a tropoelastin composition comprising a concentration of 0.5 mg/ml to 200 mg/ml tropoelastin into skin tissue within the treatment area so as to enable elastic fibre formation in the treatment area, wherein at least one of;
i) the tropelastin composition is injected into skin tissue within the treatment area according to a predetermined treatment schedule, wherein said predetermined treatment schedule is **characterized by** having one or more treatments, each treatment in the form of at least one injection to the treatment area, and wherein at least one treatment of the treatment schedule includes multiple injections to the treatment area to establish an amount of tropoelastin within the treatment area that is increased relative to skin outside the treatment area; and
ii) the tropelastin composition is injected into skin tissue within the treatment area to establish an amount of tropoelastin within the treatment area that is increased relative to skin outside the treatment area, and wherein the tropoelastin composition is adapted for sustained release of tropoelastin in skin tissue within the treatment area,
wherein the tropoelastin composition comprises tropoelastin and hyaluronic acid, and wherein the tropoelastin is substantially devoid of intramolecular cross-links,
thereby maintaining an amount of tropoelastin in the treatment area to enable elastic
fibre formation and thereby improving the appearance of the skin of the individual, wherein improving the appearance of skin is at least one of
a) improving the appearance of a stretch mark in the individual;
b) minimising the degeneration of an elastic profile of a skin tissue of the individual;
c) improving the elastic profile and/or physical appearance of aged tissue in the individual; and
d) increasing the thickness of skin while maintaining or improving the skin elasticity in the individual.

2. The method according to claim 1 wherein each treatment of the treatment schedule is spaced part by 1 to 24 weeks.

3. The method according to any one of claims 1 or 2, wherein when a treatment includes multiple injections to the treatment area, each injection is made at an injection site that is spaced apart from other injection sites by 1 mm to 3 cm.

4. The method according to any one of claims 1 to 3 wherein the tropoelastin composition is injected in a volume of 10 µl to 100 µl.

5. The method according to any one of the preceding claims wherein the tropoelastin is linked to hyaluronic acid, and wherein more preferably the composition includes from 0.5 to 50 mg/ml tropoelastin and 0.1% to 1% hyaluronic acid.

6. The method according to any one of claims 1 to 5, wherein the composition consists of tropoelastin, hyaluronic acid and an aqueous solution.

7. The method according to any one of claims 1 to 6 wherein the treatment schedule includes injections into the treatment area every 2 to 8 weeks, optionally wherein the treatment schedule spans a period of about 1 to 6 months, preferably about 1 to 3 months.

8. The method according to claim 1 wherein the treatment area includes a stretch mark and the tropoelastin composition is injected within the stretch mark.

9. The method according to claim 1, wherein the treatment area includes scarred or fibrotic tissue.

## Patentansprüche

1. Nicht-therapeutisches Verfahren zur Verbesserung des Aussehens der Haut eines Individuums, wobei das Verfahren die folgenden Behandlungsschritte umfasst:
- Bereitstellen eines Individuums;
- Definieren eines Behandlungsbereichs auf der Haut des Individuums, wobei der Behandlungsbereich ein Hautbereich ist, der eine Verbesserung des Aussehens erfordert;
- Injizieren einer Tropoelastin-Zusammensetzung mit einer Konzentration von 0,5 mg/ml bis 200 mg/ml Tropoelastin in das Hautgewebe innerhalb des Behandlungsbereichs, um die Bildung elastischer Fasern in dem Behandlungsbereich zu ermöglichen, wobei mindestens eines von:
i) die Tropoelastin-Zusammensetzung in das Hautgewebe innerhalb des Behandlungsbereichs nach einem vorbestimmten Behandlungsplan injiziert wird, wobei der vorbestimmte Behandlungsplan **dadurch gekennzeichnet ist, dass** er eine oder mehrere Behandlungen umfasst, wobei jede Behandlung in Form von mindestens einer Injektion in den Behandlungsbereich erfolgt, und wobei mindestens eine Behandlung des Behandlungsplans mehrere Injektionen in den Behandlungsbereich umfasst, um eine Menge an Tropoelastin innerhalb des Behandlungsbereichs zu etablieren, die relativ zur Haut außerhalb des Behandlungsbereichs erhöht ist, und
ii) die Tropoelastin-Zusammensetzung in das Hautgewebe innerhalb des Behandlungsbereichs injiziert wird, um eine Menge an Tropoelastin innerhalb des Behandlungsbereichs zu etablieren, die relativ zur Haut außerhalb des Behandlungsbereichs erhöht ist, und wobei die Tropoelastin-Zusammensetzung für eine anhaltende Freisetzung von Tropoelastin im Hautgewebe innerhalb des Behandlungsbereichs adaptiert ist,
wobei die Tropoelastin-Zusammensetzung Tropoelastin und Hyaluronsäure umfasst, und wobei das Tropoelastin im Wesentlichen frei von intramolekularen Vernetzungen ist,
wodurch eine Menge an Tropoelastin in dem Behandlungsbereich aufrechterhalten wird, um eine elastische Faserbildung zu ermöglichen und dadurch das Aussehen der Haut des Individuums zu verbessern, wobei die Verbesserung des Aussehens der Haut mindestens eines der folgenden Merkmale ist
a) Verbesserung des Aussehens von Dehnungsstreifen bei der Person,
b) Minimierung der Degeneration der elastischen Profile des Hautgewebes des Individuums,
c) Verbesserung des elastischen Profils und/oder des physischen Aussehens von gealtertem Gewebe bei dem Individuum, und
d) Erhöhung der Hautdicke unter Beibehaltung oder Verbesserung der Hautelastizität des Individuums.

2. Verfahren nach Anspruch 1, wobei jede Behandlung des Behandlungsplans in einem Abstand von 1 bis 24 Wochen erfolgt.

3. Verfahren nach einem der Ansprüche 1 oder 2, wobei, wenn eine Behandlung mehrere Injektionen in den Behandlungsbereich umfasst, jede Injektion an einer Injektionsstelle vorgenommen wird, die von anderen Injektionsstellen um 1 mm bis 3 cm beabstandet ist.

4. Verfahren nach einem der Ansprüche 1 bis 3, wobei die Tropoelastin-Zusammensetzung in einem Volumen von 10 µl bis 100 µl injiziert wird.

5. Verfahren nach einem der vorhergehenden Ansprüche, bei dem das Tropoelastin mit Hyaluronsäure verknüpft ist, und wobei die Zusammensetzung vorzugsweise 0,5 bis 50 mg/ml Tropoelastin und 0,1% bis 1% Hyaluronsäure enthält.

6. Verfahren nach einem der Ansprüche 1 bis 5, wobei die Zusammensetzung aus Tropoelastin, Hyaluronsäure und einer wässrigen Lösung besteht.

7. Verfahren nach einem der Ansprüche 1 bis 6, wobei der Behandlungsplan Injektionen in den Behandlungsbereich alle 2 bis 8 Wochen umfasst, wobei der Behandlungsplan gegebenenfalls einen Zeitraum von etwa 1 bis 6 Monaten, vorzugsweise von etwa 1 bis 3 Monaten umfasst.

8. Verfahren nach Anspruch 1, wobei der Behandlungsbereich einen Dehnungsstreifen umfasst und die Tropoelastin-Zusammensetzung in den Dehnungsstreifen injiziert wird.

9. Verfahren nach Anspruch 1, wobei der Behandlungsbereich vernarbtes oder fibrotisches Gewebe einschließt.

## Revendications

1. Procédé non thérapeutique d'amélioration de l'apparence de la peau d'un individu, le procédé comprenant les étapes de traitement suivantes :
- la mise à disposition d'un individu ;
- la définition d'une zone de traitement sur la peau de l'individu, dans lequel la zone de traitement est une zone de peau dont l'apparence doit être améliorée ;
- l'injection d'une composition de tropoélastine comprenant une concentration de 0,5 mg/ml à 200 mg/ml de tropoélastine dans le tissu cutané à l'intérieur de la zone de traitement afin de permettre la formation de fibres élastiques dans la zone de traitement, dans lequel au moins un élément parmi :
i) la composition de tropoélastine est injectée dans le tissu cutané à l'intérieur de la zone de traitement selon un programme de traitement prédéterminé, dans lequel ledit programme de traitement prédéterminé est **caractérisé en ce qu'**il comprend un ou plusieurs traitements, chaque traitement se présentant sous la forme d'au moins une injection dans la zone de traitement, et dans lequel au moins un traitement du programme de traitement comprend des injections multiples dans la zone de traitement pour établir une quantité de tropoélastine à l'intérieur de la zone de traitement qui est accrue par rapport à la peau hors de la zone de traitement ; et
ii) la composition de tropoélastine est injectée dans le tissu cutané à l'intérieur de la zone de traitement pour établir une quantité de tropoélastine à l'intérieur de la zone de traitement qui est accrue par rapport à la peau à l'extérieur de la zone de traitement, et dans lequel la composition de tropoélastine est adaptée pour une libération prolongée de tropoélastine dans le tissu cutané à l'intérieur de la zone de traitement,
dans lequel la composition de tropoélastine comprend de la tropoélastine et de l'acide hyaluronique, et dans lequel la tropoélastine est sensiblement dépourvue de réticulations intramoléculaires,
maintenant ainsi une quantité de tropoélastine dans la zone de traitement pour permettre la formation de fibres élastiques et améliorant ainsi l'apparence de la peau de l'individu,
dans lequel l'amélioration de l'apparence de la peau de l'individu est au moins un élément parmi
a) l'amélioration de l'apparence d'une vergeture chez l'individu ;
b) la réduction au minimum de la dégénérescence d'un profil élastique d'un tissu cutané de l'individu ;
c) l'amélioration du profil élastique et/ou de l'apparence physique d'un tissu âgé chez l'individu ; et
d) l'augmentation de l'épaisseur de la peau tout en maintenant ou en améliorant l'élasticité de la peau chez l'individu.

2. Procédé de traitement selon la revendication 1 dans lequel chaque traitement du programme de traitement est espacé de 1 à 24 semaines.

3. Procédé selon l'une quelconque des revendications 1 ou 2, dans lequel lorsqu'un traitement comprend des injections multiples dans la zone de traitement, chaque injection est effectuée au niveau d'un site d'injection qui est espacé des autres sites d'injection de 1 mm à 3 cm.

4. Procédé selon l'une quelconque des revendications 1 à 3 dans lequel la composition de tropoélastine est injectée en un volume de 10 µl à 100 µl.

5. Procédé selon l'une quelconque des revendications précédentes dans lequel la tropoélastine est liée à de l'acide hyaluronique, et dans lequel la composition comprend plus préférablement de 0,5 à 50 mg/ml de tropoélastine et de 0,1 % à 1 % d'acide hyaluronique.

6. Procédé selon l'une quelconque des revendications 1 à 5, dans lequel la composition est constituée de tropoélastine, d'acide hyaluronique et d'une solution aqueuse.

7. Procédé selon l'une quelconque des revendications 1 à 6, dans lequel le programme de traitement comprend des injections dans la zone de traitement toutes les 2 à 8 semaines, facultativement dans lequel le programme de traitement s'étend sur une période d'environ 1 à 6 mois, de préférence d'environ 1 à 3 mois.

8. Procédé selon la revendication 1 dans lequel la zone de traitement comprend une vergeture et la composition de tropoélastine est injectée à l'intérieur de la vergeture.

9. Procédé selon la revendication 1, dans lequel la zone de traitement comprend un tissu cicatriciel ou fibrotique.
